# EUROPÄISCHE PATENTSCHRIFT

(11) **EP 1 808 154 B1**
(45) Veröffentlichungstag und Bekanntmachung des Hinweises auf die Patenterteilung: **20.02.2013**
(21) Anmeldenummer: 07000687.9
(22) Anmeldetag: 15.01.2007
(51) Int. Cl.: A61Q 9/02, A61K 8/04, A61K 8/44, A61K 8/73, A61K 8/92

(54) **Rasierschaum im Pumpspender**
Shaving foam in a pump dispenser
Mousse à raser dans un distributeur à pompe

(30) Priorität: 13.01.2006 DE 102006001754
(43) Veröffentlichungstag der Anmeldung: 18.07.2007
(73) Patentinhaber: LOGOCOS Naturkosmetik AG, 31020 Salzhemmendorf (DE)
(72) Erfinder: Weiland-Groterjahn, Heinz-Jürgen, Hameln, 31787 (DE)
(74) Vertreter: Lins, Martina

(56) Entgegenhaltungen:
- EP-A- 0 034 126
- EP-A- 1 316 300
- EP-A- 1 609 464
- WO-A-03/051319
- DE-A1- 10 148 393

## Beschreibung

Die Erfindung betrifft die Verwendung einer Mischung mit einem Tensidanteil für einen treibgasfrei in einem Schaum-Pumpspender (Foamer) aufschäumbaren Rasierschaum, auch in einem Pumpspender, der die aufzuschäumende, den Rasierschaum bildende Mischung enthält.

Seifenbasierte und tensidische Rasierhilfen werden in verschiedenen Darreichungsformen angeboten. Klassisch ist die Rasierseife, die mit einem Rasierpinsel aufgeschäumt wird. Da dies zeit- und kraftraubend ist, kamen mit der Entwicklung von Treibgasbehältern bald für Sprühschäume geeignete Formulierungen auf, wie z. B. in DT 2 422 937 offenbart. Die Sprühschäume sind sehr bequem in der Anwendung und können sehr feinblasig und stabil erhalten werden, was gelegentlich sogar zu dem Problem führt, dass diese Schäume für die Anwendung zu stabil sind, sich daher trocken und flockig anfühlen, die Haut nicht genügend benetzen und sich nicht gut verteilen lassen. Weiterhin sind Rasiercremes in der Tube bekannt, die jedoch vor der Anwendung mit Wasser und Rasierpinsel aufgeschäumt werden müssen.

Am Markt gewünscht sind Produkte, die einen klassischen Schaum bilden. Diese Schäume sollen sich angenehm und leicht anfühlen, hautpflegende Wirkung besitzen, die Barthaare für die Rasur vorbereiten, und nicht schwer auf der Haut kleben wie Gele oder Cremes. Der Schaum soll sich außerdem mit der Klinge leicht abziehen und leicht abwaschen lassen. Nach der Rasur soll ein angenehmes und gepflegtes Hautgefühl hinterbleiben.

Marktgängige Formulierungen in Cremeform oder als Druckgasformulierungen, die Seifen, bzw. verseifte Fettsäuren enthalten, sind immer alkalisch, was dazu führt, dass die Rasierklingen schneller stumpf werden und korrodieren. Auch ist die Hautverträglichkeit nicht optimal. Außerdem wird der hauteigene pH-Wert stark verändert und die Haut stark ausgetrocknet.

Die WO 03/051319 A beschreibt eine Fülle kosmetischer Reinigungsprodukte aus ölhaltigen und dennoch transparenten Mikroemulsionen, die jeweils ein Primärtensid, ein oder mehrere Co-Tenside, ein oder mehrere Ölphasen, ggf. einen Verdicker, WO-Emulgatoren und/oder Salze enthalten, darunter einige Rasierprodukte.

Die DE 101 48 393 A1 beschreibt kosmetische Reinigungsschäume, die ein oder mehrere anionische Tenside und ein oder mehrere anionische Polymere zur Schaumstabilisierung enthalten. Als Tensid kann neben anderen Acylglutamat und/oder ein Zuckertensid verwendet werden.

Die EP-A-1316300 lehrt die Verwendung von schaumverstärkenden Tensiden, z. B. Acylglutamaten, zusammen mit einer Ölphase in kosmetischen Zusammensetzungen, die auf der Haut verbleiben, wie Hautpflegemittel und Sonnenschutzmittel. Diese Mittel können auch in treibgasfreien Pumpspendern aufgeschäumt werden.

Die EP-A-0034126 offenbart Rasierschäume, die kationische Tenside, Proteine und Feuchthaltemittel enthalten und in treibgasfreien Pumpspendern aufschäumbar sind.

Die Aufgabe der Erfindung besteht darin, einen treibgasfrei aufschäumbaren Rasierschaum auf tensidischer Basis zur Verfügung zu stellen, der eine für das Rasieren gut geeignete Konsistenz besitzt, hautpflegend und umweltfreundlich ist.

Diese Aufgabe wird durch die Verwendung einer Mischung für einen mit Hilfe eines Pumpspenders (Foamers) treibgasfrei aufzuschäumenden und abzugebenden Rasierschaum nach Anspruch 1 gelöst.

Das Produkt baut auf einer wässrigen Tensidlösung mit rein pflanzlichen Rückfettern auf, welches im pH-Wert beliebig eingestellt werden kann und sich vorzugsweise in einem hautfreundlichen, neutralen bis leicht sauren pH-Bereich befindet.

Die Tensidbasis besteht aus Acylglutamaten, die in Verbindung mit anderen anionischen oder nichtionischen Tensiden, nämlich einem Zuckertensid oder einer Mischung aus Zuckertensiden, eingesetzt werden können. Der Anteil des Zuckertensids soll dabei den der Acylglutamate nicht überschreien, d.h. dass das Zuckertensid jeweils in maximal gleicher Menge wie das Acylglutamat eingesetzt wird, vorzugsweise jedoch nicht oder nur in geringer Menge vorhanden ist.

In einer bevorzugten Ausführungsform enthält die Mischung als Tenside ausschließlich ein oder mehrere Acylglutamate, die derzeit als die verträglichsten und umweltfreundlichsten Tenside weltweit angesehen werden können. Das einzelne oder wenigstens eines der Acylglutamate des Tensidanteils ist bevorzugt ein solches mit langkettigem Acylrest, d.h. mit einer unverzweigten oder verzweigten Alkylkette von wenigstens 10 C-Atomen im Acylrest, wobei bei gemischten Acylglutamaten der überwiegende Teil langkettige Reste besitzen sollte. Geeignet ist in diesem Sinne zum Beispiel Cocoyl-Glutamat, insbesondere Natrium- und/oder DinatriumCocoyl-Glutamat.

Für die Rückfettung werden in der zu einem Rasierschaum aufschäumbaren Mischung erfindungsgemäß Öle eingesetzt, und zwar insbesondere keine Mineralöle sondern rein pflanzliche Öle wie Jojobaöl, Traubenkernöl, Mandelöl oder andere aus der Naturkosmetik bekannte pflanzliche Öle einzeln oder im Gemisch. Als Zusatzstoffe können zusätzlich noch andere pflanzliche Rückfetter eingesetzt werden, z. B. pflanzliche Fettsäuren, wie Myristinsäure, Laurinsäure, Stearinsäure oder Behensäure (nicht mit Alkalien verseift),

Als Verdickungsmittel sind natürliche Verdickungsmittel bevorzugt, insbesondere Xanthan, Carraghenan, Galactoarabinan, u.a. Verdickungsmittel oder Viskositätsgeber. Das Verdickungsmittel bewirkt in Kombination mit den Tensiden, der Öl- und der Wasserphase die Ausbildung einer Emulsion, die bei Abgabe aus dem Pumpspender aufgeschäumt wird und bei Einhalten der erfindungsgemäßen Rezeptur zu einem als Rasierschaum sehr zufriedenstellenden Schaumprodukt führt.

Außerdem stabilisieren die Verdickungsmittel zusammen mit den Tensiden die Emulsion aus der erfindungsgemäßen Mischung, die sich zwischen polarer Phase (Wasser/Glycerin/ Ethanol jeweils soweit vorhanden) und unpolarer Phase (Ölphase) in dem Pumpspender ausbildet.

Als Zusatzstoffe können sich vorzugsweise einer oder mehrere der folgenden Stoffe in der Mischung befinden:
Pflanzliche Extrakte, insbesondere als Hautpflegezusätze, wie z. B. Aloe Vera, Hamamelis, Ringelblume; Duft- oder Riechstoffe als Wirkstoffe zur Parfümierung;
Lösungsvermittler, wie insbesondere Glyceryloleate, ggf. in Verbindung mit Coco-Glucosiden oder auch in Verbindung mit Polyglycerinen, insbesondere Polyglycerin-10-laurat;
Ethanol und/oder ethanolische Biomassedestillate zur mikrobiologischen Stabilisierung, ggf. gleichzeitig als Lösungsmittel;
Feuchtigkeitsspender, insbesondere Glykole und/oder Triole, vorzugsweise Glycerin, Sorbitol oder andere;
Emulsionsstabilisatoren, Antioxidantien;
pH-Regulatoren, wie Citronensäure oder Citrate, z.B. Trinatriumcitrat, Milchsäure oder Lactate, z.B. Natriumlactat.
von denen Einzelne oder mehrere verschiedene in unterschiedlichen Kombinationen in der Mischung eingesetzt werden können.

Glycerin wird als Zusatzstoff (Feuchtigkeitsspender) in einer Menge von 0,1 bis 10 Gew.-% bezogen auf die aufschäumbare Mischung eingesetzt. Allgemein werden die Glykole und/oder Triole, soweit vorhanden, in einem Verhältnis von 1:2 bis 2:1 bezogen auf die Gesamttensidmasse eingesetzt, vorzugsweise in ähnlicher Menge wie diese, d.h. in einem Verhältnis von ca. 1:1,2 bis 1,2:1.

Ethanol und/oder ethanolisches Biomassedestillat wird vorzugsweise in einer Menge von ca. 0,5 bis 10 Gew.-%eingesetzt, weiter vorzugsweise 0,5 bis 5 Gew.-% bezogen auf die aufschäumbare Mischung. Als ethanolisches Biomassedestillat kann bevorzugt Öko-Weizendestillat verwendet werden.

Das Öl oder die Mischung mehrerer Öle wird erfindungsgemäß in der 3- bis 5-fachen Menge des Tensidanteils der Mischung eingesetzt.

Die Mischung wird mit Wasser aufgefüllt, wobei die Inhalts- und Zusatzstoffe teilweise in wässriger Lösung zugemischt werden können. Das Wasser bildet mit den hydrophilen Inhalts- und Zusatzstoffen eine wässrige Phase, die mit den Ölen und rein fettlöslichen Bestandteilen der Mischung eine Emulsion bilden kann. Diese Emulsion wird durch die Tensid-Komponente mit Hilfe eines für das Aufschäumen geeigneten Pumpspenders aufgeschäumt. Gegebenenfalls wird die Mischung in dem Pumpspender vorher geschüttelt, um eine Emulsion zu bilden, die dann bei Abgabe durch den Pumpspender aufgeschäumt wird.

Die erfindungsgemäße Rasierschaum-Mischung entspricht den strengen Naturkosmetikkriterien des BDIH (Bundesverband der deutschen Industrie- und Handelsunternehmen).

Der erfindungsgemäße Rasierschaum hat folgende Vorteile:
- er entspricht den Standards für Naturkosmetik und ist biologisch gut abbaubar,
- er ist in einem Pumpspender (Foamer) optimal aufschäumbar und bildet einen stabilen, nicht zu leicht zerfließenden aber dennoch feuchten und gut hautbenetzenden Schaum,
- er ist gut verteilbar und fühlt sich angenehm an,
- er schont die Rasierklingen (wirkt nicht feststellbar korrosiv),
- er ist im gewünschten, i.a. neutralen bis leicht sauren pH-Bereich einstellbar und somit hautfreundlich,
- er bereitet die Barthaare gut für die Rasur vor,
- er ist rückfettend und dennoch gut abwaschbar.

Die erfindungsgemäße Rasierschaum-Formulierung liegt vorzugsweise in einem Pumpspender vor. in einem Reservoir. Das Reservoir sollte so dimensioniert, dass ein freies Volumen zum Aufschütteln vorhanden ist.

Bei dem Pumpspender an sich kann es sich um einen handelsüblichen Pumpspender für die Abgabe von Schäumen handeln (Foamer). Derartige Pumpspender bestehen im Allgemeinen aus einer Pumpspenderflasche, bzw. einem beliebig geformten Pumpspender-Behälter, der oder die mit einem schaumbildenden Abgabekopf versehen ist. Vom Abgabekopf ausgehend reicht ein Steigrohr in die Flasche oder den Behälter hinein, so dass dieses Rohr möglichst in jedem Füllungszustand in die aufzuschäumende Flüssigkeit oder Emulsion eintaucht. Der Abgabekopf umfasst weiterhin wenigstens ein Ventil mit Abgabedüse und eine Druckaufgabeeinrichtung, mit der von Hand durch im Allgemeinen mehrfaches betätigen der Einrichtung Druck auf den Behälter gegeben wird, um den Inhalt in Portionen heraus zu treiben und dabei aufzuschäumen. Bei den meisten Pumpspendern ist vorheriges Schütteln erforderlich, was jedoch von der Art der Schaumdüse abhängig ist.

Solche Foamer sind im Handel erhältlich. Beispiele für schaumabgebende Pumpspender finden sich beispielsweise in der US 6,053,365 oder der EP 613728.

Die erfindungsgemäße Rasierschaum-Formulierung ist für die Verwendung in treibgasfrei betriebenen Pumpspender optimiert und führt zu Schäumen, die die an Rasierschäume zu stellenden Bedingungen gut erfüllen.

Im Folgenden wird die Erfindung anhand einiger Beispiele näher illustriert, die jedoch lediglich dem besseren praktischen Verständnis der Erfindung und nicht etwa einer Beschränkung der Erfindung auf diese Beispiele dienen sollen.

### BEISPIELE

Es werden tabellarisch Rezepturen für Mischungen angegeben, die in geeigneten, handelsüblichen Pumpspendern (Foamern) zu Rasierschäumen aufschäumbar sind.

### Beispiel 1

| | Anteil in Gew.-% | Inhaltsstoff |
|---|---|---|
| Tensidanteil | 3,75 | Acylglutamat |
| Ölkomponente | 15,3 | pflanzliche Öle, Mischung aus: Traubenkernöl, Jojobaöl, Mandelöl |
| Verdickungsmittel | 0,4 | Xanthan |
| | 0,5 | Galactoarabinan |
| Zusatzstoff: Pflegeextrakt | 5,0 | Mischung aus: Aloe Vera Saft, Hammamelisextrakt |
| Zusatzstoff: Alkohol | 4,0 | Öko-Weizeldestillat |
| Zusatzstoff: Parfümierung | 1,2 | Duftstoffe |
| Zusatzstoff: Feuchtigkeitsspender | 4,0 | Glycerin |
| | 65,85 | Wasser |

### Beispiel 2

| | Anteil in Gew.-% | Inhaltsstoff |
|---|---|---|
| Tensidanteil | 3,7 | Acylglutamat |
| Ölkomponente | 13,3 | pflanzliche Öle, Mischung aus: Traubenkernöl, Jojobaöl, Mandelöl |
| Verdickungsmittel | 0,3 | Xanthan |
| | 0,5 | Galactoarabinan |
| Zusatzstoff: Pflegeextrakt | 5,0 | Mischung aus: Aloe Vera Saft, Hammamelisextrakt |
| Zusatzstoff: Parfümierung | 1,45 | Duftstoffe |
| Zusatzstoff: Feuchtigkeitsspender | 4,0 | Glycerin |
| | 71,75 | Wasser |

### Beispiel 3

| | Anteil in Gew.-% | Inhaltsstoff |
|---|---|---|
| Tensidanteil | 3,7 | Acylglutamat |
| Ölkomponente | 13,3 | pflanzliche Öle, Mischung aus: Traubenkernöl, Jojobaöl, Mandelöl |
| Verdickungsmittel | 0,3 | Xanthan |
| | 0,5 | Galactoarabinan |
| Zusatzstoff: Pflegeextrakt | 5,0 | Mischung aus: Aloe Vera Saft, Hammamelisextrakt |
| Zusatzstoff: Alkohol | 1,0 | Öko-Weizeldestillat |
| Zusatzstoff: Parfümierung | 1,46 | Duftstoffe |
| Zusatzstoff: Feuchtigkeitsspender | 4,0 | Glycerin |
| | 70,74 | Wasser |

## Patentansprüche

1. Verwendung einer Mischung bestehend aus
einem Tensidanteil von maximal 5 Gew.-%, wobei der Tensidanteil aus wenigstens einem Acylglutamat und optional zusätzlich wenigstens einem anderen anionischen oder nichtionischen Tensid, nämlich einem Zuckertensid, in maximal gleicher Menge besteht,
2 bis 25 Gew.-% eines Öls oder einer Mischung mehrerer Öle, wobei das Öl oder die Mischung mehrerer Öle in der 3- bis 5-fachen Menge des Tensidanteils der Mischung eingesetzt wird/werden und das oder die Öle ausgewählt sind aus pflanzlichen Ölen,
0,1 bis 2 Gew.-% eines Verdickungsmittels
0,1 bis 10 Gew.-% Glycerin und gegebenenfalls weitere Feuchtigkeitsspender bis zu einem Gesamtgehalt von 20 Gew.-%,
je 0 bis 20 Gew.-% weiterer Zusatzstoffe, ausgewählt aus der Gruppe bestehend aus: pflanzlichen Extrakten, insbesondere als Hautpflegezusätze, Duft- und Riechstoffen, Lösungsvermittlern, Ethanol und/oder ethanolischen Blomassedestillaten, Emulsionsstabilisatoren, Antioxidantlen, pH-Regulatoren, von denen Einzelne oder mehrere verschiedene in der Mischung eingesetzt werden können,
ad 100 % Wasser, für einen mit Hilfe eines Pumpspenders treibgasfrei aufzuschäumenden und abzugebenden Rasierschaum.

2. Verwendung nach Anspruch 1, **dadurch gekennzeichnet, dass** das Verdickungsmittel ein natürliches Verdickungsmittel ist.

3. Verwendung nach Anspruch 1 oder 2, **dadurch gekennzeichnet, dass** das einzelne oder wenigstens eines der Acylglutamate des Tensidanteils ein solches mit langkettigem Acylrest ist, vorzugsweise ein Cocoyl-Glutamat, insbesondere Natrium- und/oder Dinatrium-Cocoly-Glutamat.

4. Verwendung nach einem der Ansprüche 1 bis 3, **dadurch gekennzeichnet, dass** Ethanol und/oder alkoholisches Biomassedestillat in einer Menge von 0,5 bis 10 Gew.-%, weiter vorzugsweise 0,5 bis 5 Gew.-% bezogen auf die Mischung eingesetzt wird.

5. Verwendung gemäß einem der Ansprüche 1 bis 4 in einem Pumpspender mit Reservoir für die Mischung.

6. Verwendung nach Anspruch 5, **dadurch gekennzeichnet, dass** das Reservoir so dimensioniert ist, dass ein freies Volumen zum Aufschütteln der aufschäumbaren Mischung vorhanden ist.

## Claims

1. Use of a mixture consisting of
a surfactant component of not more than 5% by weight, where the surfactant component is composed of at least one acylglutamate and optionally additionally at least one other anionic or nonionic surfactant, which is a sugar surfactant, in not more than an equal amount,
2 to 25% by weight of an oil or of a mixture of a plurality of oils, where the oil or the mixture of a plurality of oils is/are employed in a 3- to 5-fold amount of the surfactant component of the mixture and the oil(s) is/are selected from among vegetable oils,
0.1 to 2% by weight of thickener,
0.1 to 10% by weight of glycerol and optionally other humectants up to a total content of 20% by weight,
in each case 0 to 20% by weight of other additives, selected from the group consisting of: plant extracts, in particular as skin care additives, fragrances and other odoriferous substances, solubilizers, ethanol and/or ethanolic biomass distillates, emulsion stabilizers, antioxidants, pH regulators, of which individual ones or a plurality of different ones may be employed in the mixture,
water to 100%, for a shaving foam which can be foamed and dispensed with the aid of a pump dispenser, without the use of propellants.

2. Use according to Claim 1, **characterized in that** the thickener is a natural thickener.

3. Use according to Claim 1 or 2, **characterized in that** the individual, or at least one of the, acylglutamates of the surfactant component is one with a long-chain acyl radical, preferably a cocoyl-glutamate, in particular sodium and/or disodium cocoyl-glutamate.

4. Use according to any one of Claims 1 to 3, **characterized in that** ethanol and/or alcoholic biomass distillate is employed in an amount of from 0.5 to 10% by weight, more preferably 0.5 to 5% by weight, based on the mixture.

5. Use according to any of Claims 1 to 4 in a pump dispenser with a reservoir for the mixture.

6. Use according to Claim 5, **characterized in that** the dimensions of the reservoir are such that a free volume is available for shaking up the foamable mixture.

## Revendications

1. Utilisation d'un mélange consistant en
une fraction tensioactive d'au maximum 5 % en poids, la fraction tensioactive consistant en au moins un acylglutamate et en option additionnellement en au moins un autre tensioactif anionique ou non ionique, à savoir un tensioactif glucidique, au maximum en la même quantité,
2 à 25 % en poids d'une huile ou d'un mélange de plusieurs huiles, l'huile ou le mélange de plusieurs huiles étant utilisé(e) en la quantité triple à quintuple de la fraction tensioactive du mélange et l'huile ou les huiles étant choisies parmi des huiles végétales,
0,1 à 2 % en poids d'un épaississant,
0,1 à 10 % en poids de glycérol et éventuellement d'autres hydratants jusqu'à une concentration totale de 20 % en poids,
0 à 20 % en poids chacun d'autres additifs, choisis dans le groupe constitué par : des extraits végétaux, en particulier en tant qu'additifs pour le soin de la peau, des parfums et substances odorantes, des tiers-solvants, l'éthanol et/ou des distillats éthanoliques de biomasse, des stabilisants d'émulsion, des antioxydants, des régulateurs du pH, dont des représentants individuels ou plusieurs représentants différents peuvent être utilisés dans le mélange, en complément à 100 % en eau, pour une mousse de rasage à libérer et faire mousser sans gaz propulseur à l'aide d'un distributeur à pompe.

2. Utilisation selon la revendication 1, **caractérisée en ce que** l'épaississant est un épaississant naturel.

3. Utilisation selon la revendication 1 ou 2, **caractérisée en ce que** l'unique ou au moins l'un des acylglutamate(s) du tensioactif est un acylglutamate à radical acyle à longue chaîne, de préférence un cocoyl-glutamate, en particulier le cocoylglutamate de sodium ou disodique.

4. Utilisation selon l'une quelconque des revendications 1 à 3, **caractérisée en ce que** l'éthanol et/ou le distillat de biomasse alcoolique est contenu en une quantité de 0,5 à 10 % en poids, encore mieux de 0,5 à 5 % en poids, par rapport au mélange.

5. Utilisation selon l'une quelconque des revendications 1 à 4, dans un distributeur à pompe à réservoir pour le mélange.

6. Utilisation selon la revendication 5, **caractérisée en ce que** le réservoir est dimensionné de sorte qu'un volume libre soit présent pour le secouement du mélange transformable en mousse.
